# EUROPÄISCHE PATENTSCHRIFT

(11) **EP 3 380 446 B1**
(45) Veröffentlichungstag und Bekanntmachung des Hinweises auf die Patenterteilung: **05.02.2020**
(21) Anmeldenummer: 16819878.6
(22) Anmeldetag: 17.12.2016
(51) Int. Cl.: C05C 9/00, C07C 273/14, C05G 3/00, B01J 2/30, B01J 2/16, A23K 40/10, A23K 20/10, A23K 50/15

(54) **HARNSTOFFHALTIGE DÜNGEMITTEL UND VERFAHREN ZU IHRER HERSTELLUNG**
UREA-CONTAINING FERTILIZERS AND PROCESS FOR PRODUCTION THEREOF
ENGRAIS CONTENANT DE L'URÉE ET PROCÉDÉ POUR SA PRÉPARATION

(30) Priorität: 17.12.2015 DE 102015122070
(43) Veröffentlichungstag der Anmeldung: 03.10.2018
(73) Patentinhaber: thyssenkrupp Fertilizer Technology GmbH, 44141 Dortmund (DE); thyssenkrupp AG, 45143 Essen (DE)
(72) Erfinder: POTTHOFF, Matthias, 44265 Dortmund (DE); KRAWCZYK, Thomas, 42117 Wuppertal (DE); FRANZRAHE, Harald, 44289 Dortmund (DE); VANMARCKE, Luc Albert, 9971 Lembecke (BE)
(74) Vertreter: thyssenkrupp Intellectual Property GmbH
(86) Internationale Anmeldenummer: PCT/EP2016/081626
(87) Internationale Veröffentlichungsnummer: WO 2017/103243

(56) Entgegenhaltungen:
- EP-A2- 0 533 024
- WO-A1-2006/091077
- WO-A1-2015/193377
- FR-A- 1 021 100
- FR-A1- 2 874 008
- US-A1- 2004 009 878

## Beschreibung

Die Erfindung betrifft eine partikelförmige, Harnstoff-haltige Zusammensetzung und die Verwendung eines Additivs zur Herstellung einer partikelförmigen, Harnstoff-haltigen Zusammensetzung.

Für die Herstellung von partikelförmigen, Harnstoff-haltigen Zusammensetzungen sind verschiedene Verfahren im Stand der Technik bekannt. In der Vergangenheit wurden Harnstoffpartikel üblicherweise mittels Sprühkristallisation hergestellt, wobei eine im wesentlichen wasserfreie Harnstoffschmelze (Wassergehalt von 0,1 bis 0,3 Gew.-%) vom oberen Teil eines Sprühkristallisationsturmes in einen aufsteigenden Strom von Luft bei Umgebungstemperatur gesprüht werden und sich die Tropfen zu Kristallen (Prills) verfestigen. Die so erhaltenen Prills weisen relativ kleine Durchmesser sowie eine geringe mechanische Festigkeit auf.

Weitere bekannte Verfahren zur Herstellung von partikelförmigen, Harnstoff-haltigen Zusammensetzungen verwenden Trommel- oder Tellergranulieranlagen, als auch sogenannte Spherodizertrommeln zur Herstellung der Partikel.

Harnstoffpartikel mit größeren Teilchendurchmessern und besseren mechanischen Eigenschaften werden heute zumeist durch Granulierung einer im wesentlichen wasserfreien Harnstoffschmelze oder einer wässrigen Harnstofflösung in einem Fließbett hergestellt, wie beispielsweise in der US 4,219,589 beschrieben. In diesen Granulierungsverfahren wird eine wässrige Harnstofflösung mit einer Harnstoffkonzentration von 70-99,9 Gew.-% in Form von sehr fein verteilten Tröpfchen mit einem durchschnittlichen Durchmesser von 20-120 µm in ein Fließbett von Harnstoffpartikeln gegeben, wobei die Temperatur so gewählt ist, dass das Wasser der auf die Harnstoffpartikel aufgesprühten Lösung verdampft und Harnstoff auf den Partikeln abgeschieden wird, so dass ein Granulat mit einer gewünschten Partikelgröße von 2,5 mm und mehr erhalten wird.

Da in diesem Verfahren relativ große Mengen an feinem Staub anfallen, insbesondere wenn die eingesetzte Harnstofflösung einen Wassergehalt von mehr als 5 Gew.-% aufweist, werden häufig Granulierungsadditive eingesetzt, die diese Staubbildung reduzieren. Die Zugabe dieser Additive führt dazu, dass die Granulatpartikel und insbesondere deren Oberfläche plastisch bleiben, so dass infolge ihrer Rollbewegungen und Zusammenstöße runde Partikel mit einer glatten Oberfläche und einer guten mechanischen Stabilität erhalten werden.

Das so erhaltene Granulat weist daher eine hohe Druck- und Stoßfestigkeit auf, eine geringe Tendenz zur Staubbildung durch Abrieb sowie darüber hinaus selbst bei längerer Lagerung nur eine geringe Neigung zur Verklumpung. Entsprechende Granulierungsadditive finden aber nicht nur in der Fließbettgranulierung ihre Anwendung, sondern auch in anderen oben erwähnten Verfahren.

Als Granulierungsadditive werden üblicherweise Formaldehyd bzw. wasserlösliche Additions- und/oder Kondensationsprodukte aus Formaldehyd und Harnstoff eingesetzt, die aber in relativ großen Mengen zugegeben werden müssen und aufgrund ihrer toxischen Eigenschaften in ihrer Handhabung nicht unproblematisch sind. Austritte von Formaldehyd stellen ein akutes Gesundheits- und Umweltrisiko dar, obwohl die Einführung von Harnstoff-Formaldehyd Präpolymeren wie UF80 oder UF85 derartige Risiken verringert hat. Zudem stellt sich die Frage von Gesundheitsrisiken auch im Zusammenhang mit der chronischen Exposition gegenüber Formaldehyddämpfen, die auch durch die Verwendung derartiger Präpolymere nicht gänzlich vermieden werden.

Ein weiteres Problem bei der Granulierung von Harnstoff-haltigen Partikeln stellt die Entstehung von Staub dar, wobei hierunter Partikel mit einem Durchmesser von weniger als 0,5 mm verstanden werden. Diese Entstehung von Staub ist im Wesentlichen auf drei Quellen zurückzuführen. Zunächst ist der Abrieb des Granulats aufgrund von Bewegungen und Zusammenstößen der Partikel zu nennen, beispielsweise im Fließbett, wobei die Menge des anfallenden Staubs wesentlich von den mechanischen Eigenschaften des Granulats abhängt. Des Weiteren erzeugen die Düsen bzw. die in den anderen Verfahren verwendeten Flüssigkeitsverteiler jeweils Tropfen mit einer gewissen Verteilung an Durchmessern, wobei sich die feinsten Tropfen dann verfestigen bevor sie auf die Harnstoffpartikel treffen, so dass der so gebildete Staub den Granulator mit der Abluft wieder verlässt. Schließlich ist als dritte Quelle der aus der Zerkleinerung zu großer Granulatpartikel gewonnene Staub zu nennen, der in den Verfahren und Anlagen nach dem Stand der Technik üblicherweise direkt wieder in den Granulator überführt wird. 10 bis 20 Gew.-% der zerkleinerten Partikel haben einen Durchmesser von weniger als 1 mm und ein großer Anteil davon ist Staub. So werden über diesen Anteil an zerkleinerten Partikel 1 bis 1,5 % Staub pro Tonne des Endproduktes wieder in den Granulator zurückgeführt und 3-5 % des Gesamtstaubes pro Tonne des Endproduktes einer Industrieanlage sind auf den Granulator zurückzuführen.

Zur Vermeidung bzw. Reduzierung der vorstehend genannten Nachteile wurden unterschiedliche Alternativen zu Formaldehyd und dessen wasserlöslichen Additions- und/oder Kondensationsprodukten untersucht, die aber jeweils auch mit Einschränkungen bzw. Nachteilen behaftet sind.

Beispielhaft sei auf die Verwendung von Alkalimetalllignosulfonaten verwiesen, wie in der US 5,766,302 beschrieben oder auf die Verwendung von Glyoxal oder Kohlenhydraten. Diese führen aber in dem erhaltenen Harnstoffprodukt in Abhängigkeit von dem Herstellungsverfahren zu einer gelblichen oder bräunlichen Färbung, die in vielen Fällen nicht erwünscht ist. Auf der anderen Seite führt die Verwendung von oberflächenaktiven Substanzen wie beispielsweise Mischungen aus Polyvinylacetat und Polyvinylalkohol als Granulierungsadditive ebenfalls zu Problemen, da diese zum Schäumen neigen, beispielsweise wenn das Additiv mit der Schmelze gemischt wird oder in den Wäschern, wo der behandelte Harnstoffstaub aufgelöst wird und den Wirkungsgrad der Wäscher beeinträchtigt. Die Neigung dieser Substanzen zur Schaumbildung hat zudem auch Auswirkungen auf das Endprodukt, das eine geringere Dichte aufweist und vom Markt nicht akzeptiert wird. Insgesamt ist daher eine Neigung zur Schaumbildung bei industrieller Anwendung der Harnstoffgranulate nicht akzeptabel.

FR 2 874 008 A1 offenbart Dünger Granulate mit einem wasserlöslichen Polymer als Binder. US 2004/009878 A1 offenbart wasserlösliche Partikel zur Bereitstellung von Stickstoff für Pflanzen. WO 2006/091077 A1 offenbart farbechte Harnstoffzusammensetzungen mit mindestens zwei biologisch abbaubaren Polymeren. FR 1 021 100 A offenbart ein Verfahren zur Herstellung von kristallinen Harnstoff.

Es ist daher eine Aufgabe der vorliegenden Erfindung, ein Verfahren zur Herstellung von partikelförmigen Harnstoff-haltigen Zusammensetzungen zur Verfügung zu stellen, welche die Nachteile des Standes der Technik nicht oder zumindest in verringerter Form aufweist.

Diese Aufgabe wird durch den Gegenstand der Beschreibung und der Patentansprüche gelöst.

Es wurde überraschend gefunden, dass unter Verwendung bestimmter Additive eine Harnstoff-haltige, partikuläre Zusammensetzung mit zufriedenstellenden Eigenschaften ohne den Einsatz von Formaldehyd und Harnstoff-Formaldehyd Kondensaten erhalten werden kann. Insbesondere können so
- die mit dem Einsatz von Formaldehyd und Harnstoff-Formaldehyd Kondensaten verbundenen Gesundheits- und Umweltrisiken vermieden werden; und/oder
- eine kostengünstigere Alternative zur Herstellung der Zusammensetzungen gegenüber von unter Verwendung von Formaldehyd und Harnstoff-Formaldehyd Kondensaten hergestellten Zusammensetzungen bereitgestellt werden; und/oder
- die unerwünschte Färbung der Zusammensetzung reduziert oder sogar vollständig vermieden werden; und/oder
- das unerwünschte Schäumen während der Herstellung oder der Nasswäsche reduziert oder sogar vollständig vermieden werden; und/oder
- ein zu der Verwendung von Formaldehyd und Harnstoff-Formaldehyd Kondensaten vergleichbares Wachstum der Partikel erzielt werden; und/oder
- die Bildung von Staub während der Herstellung der Zusammensetzung reduziert oder sogar vollständig vermieden werden, und/oder
- eine partikelförmige Zusammensetzung erhalten werden, deren Teilchen im Vergleich zu unter der Verwendung von Formaldehyd und Harnstoff-Formaldehyd Kondensaten hergestellten Zusammensetzungen zumindest vergleichbare oder sogar bessere Eigenschaften zeigen, insbesondere in Hinblick auf mechanische Eigenschaften wie beispielsweise Druckfestigkeit, Stoßfestigkeit, geringe Neigung zum Abrieb oder zum Verklumpen, insbesondere bei längerer Lagerung.

Eine Offenbarung der Erfindung betrifft eine partikelförmige Zusammensetzung enthaltend
(i) Harnstoff;
und ein Additiv umfassend eine oder beide der Komponenten (ii) und (iii):
(ii) Kombination aus wenigstens einem Amino-Gruppen enthaltenden Polymer oder Oligomer und wenigstens einer funktionalisierten Polyvinylverbindung;
(iii) wenigstens einen aliphatischen C₂₋C₈ Dialdehyd;
und einen Zusatzstoff umfassend eine oder mehrere der Komponenten (iv) bis (vi):
(iv) Schwefel;
(v) Ammonsulfat;
(vi) mindestens ein Spurenelement;
wobei der Gewichtsanteil der Komponente (i) > 10 Gew.-% und der Gewichtsanteil der Summe der Komponenten (ii) und (iii) in der Zusammensetzung < 1 Gew.-% beträgt.

Eine weitere Offenbarung der Erfindung betrifft eine partikelförmige Zusammensetzung enthaltend
(i) Harnstoff;
und ein Additiv umfassend eine oder beide der Komponenten (ii) und (iii):
(ii) Kombination aus wenigstens einem Amino-Gruppen enthaltenden Polymer oder Oligomer und wenigstens einer funktionalisierten Polyvinylverbindung;
(iii) wenigstens einen aliphatischen C₂₋C₈ Dialdehyd;
und einen Zusatzstoff umfassend eine oder mehrere der Komponenten (iv) bis (vi):
(iv) Schwefel;
(v) Ammonsulfat;
(vi) mindestens ein Spurenelement
wobei der Gewichtsanteil der Komponente (i) > 50 Gew.-%, der Gewichtsanteil der Komponenten (iv) und (v) als Einzelkomponente oder in der Zusammensetzung < 50 Gew.-% und der Gewichtsanteil der Komponente (vi) < 5 Gew.-% und der Gewichtsanteil der Summe der Komponenten (ii) und (iii) in der Zusammensetzung < 1 Gew.-% beträgt.

In einer bevorzugten Ausführungsform beträgt der Gewichtsanteil der Komponente (i) in der partikelförmigen Zusammensetzung > 30 Gew.-%, bevorzugter 50 Gew.-%, noch bevorzugter > 60 Gew.-%, besonders bevorzugt > 70 Gew.-%, ganz besonders bevorzugt > 80 Gew.-%, noch weiter bevorzugt > 96 Gew.-%.

Bevorzugt beträgt der Gewichtsanteil der Summe der Komponenten (ii) und (iii) in der partikelförmigen Zusammensetzung < 1 Gew.-%, bevorzugter < 0,8 Gew.-%, noch bevorzugter < 0,6 Gew.-%, ganz besonders bevorzugt < 0,4 Gew.-% und insbesondere < 0,2 Gew.-%.

Umfasst der Zusatzstoff der partikelförmigen Zusammensetzung nur die Komponente (iv), beträgt der Gewichtsanteil der Komponente (iv) in der partikelförmigen Zusammensetzung bevorzugt < 50 Gew.-%, bevorzugter < 40 Gew.-%, noch bevorzugter < 30 Gew.-%, besonders bevorzugt < 20 Gew.-% und insbesondere < 10 Gew.-%. Umfasst der Zusatzstoff der partikelförmigen Zusammensetzung nur die Komponente (iv), liegt der Gewichtsanteil der Komponente (iv) in der partikelförmigen Zusammensetzung bevorzugt im Bereich von 1 bis 30 Gew.-%, bevorzugter im Bereich von 2 bis 20 Gew.-% und insbesondere im Bereich von 3 bis 10 Gew.-%.

Umfasst der Zusatzstoff der partikelförmigen Zusammensetzung nur die Komponente (v), beträgt der Gewichtsanteil der Komponente (v) in der partikelförmigen Zusammensetzung bevorzugt < 50 Gew.-%, bevorzugter < 40 Gew.-%, noch bevorzugter < 30 Gew.-%, besonders bevorzugt < 20 Gew.-% und insbesondere < 10 Gew.-%.

Umfasst der Zusatzstoff der partikelförmigen Zusammensetzung sowohl die Komponente (iv) als auch die Komponente (v), beträgt der Gewichtsanteil der Summe der Komponenten (iv) und (v) in der partikelförmigen Zusammensetzung < 50 Gew.-%, bevorzugter < 40 Gew.-%, noch bevorzugter < 30 Gew.-%, besonders bevorzugt < 20 Gew.-% und insbesondere < 10 Gew.-%.

Umfasst der Zusatzstoff der partikelförmigen Zusammensetzung die Komponente (vi), beträgt der Gewichtsanteil der Komponente (vi) in der partikelförmigen Zusammensetzung bevorzugt < 5 Gew.-%, bevorzugter < 4 Gew.-%, noch bevorzugter < 3 Gew.-%, besonders bevorzugt < 2 Gew.-% und insbesondere < 1 Gew.-%.

Erfindungsgemäß zum Einsatz kommende Amino-Gruppen enthaltende Polymere und Oligomere umfassen insbesondere Polymere und Oligomere mit einem Molekulargewicht (MW) von 250 bis 2,000,000, von 300 bis 2,000,000, von 350 bis 2,000,000, von 400 bis 2,000,000, von 450 bis 2,000,000, von 500 bis 2,000,000, von 550 bis 2,000,000, von 600 bis 2,000,000, von 650 bis 2,000,000, von 700 bis 2,000,000, von 750 bis 2,000,000, von 800 bis 2,000,000, von 850 bis 2,000,000, von 900 bis 2,000,000, von 950 bis 2,000,000, von 1000 bis 2,000,000, von 1050 bis 2,000,000, von 1100 bis 2,000,000, von 1150 bis 2,000,000, sowie von 1200 bis 2,000,000 Dalton.

Beispielsweise können die erfindungsgemäß zum Einsatz kommenden Amino-Gruppen enthaltenden Polymere und Oligomere ein Molekulargewicht (MW) von 500 bis 1,000,000, von 550 bis 1,000,000, von 600 bis 1,000,000, von 650 bis 1,000,000, von 700 bis 1,000,000, von 750 bis 1,000,000, von 800 bis 1,000,000, von 850 bis 1,000,000, von 900 bis 1,000,000, von 950 bis 1,000,000, von 1000 bis 1,000,000, von 1050 bis 1,000,000, von 1100 bis 1,000,000, von 1150 bis 1,000,000, sowie von 1200 bis 1,000,000 Dalton oder im Bereich von 500 bis 10,000, von 550 bis 10,000, von 600 bis 10,000, von 650 bis 10,000, von 700 bis 10,000, von 750 bis 10,000, von 800 bis 10,000, von 850 bis 10,000, von 900 bis 10,000, von 950 bis 10,000, von 1000 bis 10,000, von 1050 bis 10,000, von 1100 bis 10,000, von 1150 bis 10,000, sowie von 1200 bis 10,000 Dalton aufweisen.

Vorzugsweise können die Amino-Gruppen enthaltenden Polymere und Oligomere einen Stickstoffgehalt von 10 bis 50 Gew.-%, bezogen auf das Gewicht des Polymers oder Oligomers, aufweisen und primäre, sekundäre oder tertiäre Aminogruppen enthalten, die unabhängig voneinander Alkyl oder Arylalkyl-Gruppen enthalten, beispielsweise C₁₋₆-Alkyl oder Aryl-C₁₋₃-alkyl, wobei Aryl insbesondere für Phenyl oder Pyridyl stehen kann, welches unsubstituiert oder ggf. mit 1, 2, 3, 4 oder 5 Substituenten unabhängig voneinander ausgewählt aus der Gruppe bestehend aus F, Cl, Br, CF₃, C₁₋₆-Alkyl, C₁₋₆-Alkoxy, NH₂, C₁₋₆-Alkyl amino und Di(C₁₋₆-Alkyl)amino substituiert sein kann.

Beispielsweise kommen als Amino-Gruppen enthaltende Polymere und Oligomere Polyamine, polymere Polyamine, Stickstoff-substituierte Vinylpolymere, Polyoxazoline, Polypropylenimin und dessen Dendrimere, Polyethylenimin und dessen Dendrimere, Polyamidoamin und dessen Dendrimere, sowie Copolymere und Derivate und Kombinationen aus zwei oder mehr der genannten Substanzen in Betracht.

Bevorzugte Amino-Gruppen enthaltende Polymere und Oligomere umfassen Polyamine und polymere Polyamine, Polyalkylenimine wie z.B. Polyethylenimine und Polypropylenimine, Polyvinylamine, polyalkoxylierte Polyamine, ethoxylierte Polyamine, propoxylierte Polyamine, alkylierte und benzylierte Polyamine sowie Kombinationen aus zwei oder mehr der vorstehend genannten Komponenten.

Ganz besonders bevorzugt kommen als Amino-Gruppen enthaltende Polymere und Oligomere Polyethylenimine, Polyethylenimin Dendrimere, sowie deren Copolymere, Derivate und Mischungen aus wenigstens zwei dieser Komponenten zum Einsatz.

Geeignete Polyethylenimine können lineare oder verzweigte Polyethylenimin Polymere oder Oligomere umfassen mit beispielsweise 10 oder mehr Monomer-Einheiten sowie deren Derivate, Analoga, Copolymere und Mischungen aus wenigstens zwei dieser Komponenten.

Polyethylenimine können durch die Polymerisation von Ethylenimin erhalten werden und sind käuflich am Markt erhältlich, beispielsweise in Form der Lupasol® und Epomin® Produktfamilien und hier insbesondere der Produkte Lupasol® G20, Lupasol® FG, Lupasol® G35, Lupasol® P, und Lupasol®1595 (die Lupasol® Produkte sind erhältlich von der BASF (Florham Park, NJ, USA)), sowie Epomin® SP-003, Epomin® SP-006, Epomin® SP-012, Epomin® SP-018, Epomin® SP-200, Epomin® SP-1000, und Epomin® SP-1050 (die Epomin® Produkte sind erhältlich von Nippon Shokubai (Osaka, Japan)).

Als funktionalisierte Polyvinylverbindungen kommen erfindungsgemäß insbesondere Verbindungen basierend auf der Wiederholeinheit (CHXCHY)ₙ in Betracht, worin X ausgewählt ist aus der Gruppe bestehend aus H, NH₂, OH, COOH, COR, CONH₂, CH₂NH₂, CH₂NHR, CH₂OH und CH₂OR und Y ausgewählt ist aus der Gruppe bestehend aus NH₂, OH, COOH, COR, CONH₂, CH₂NH₂, CH₂NHR, CH₂OH und CH₂OR und wobei R jeweils unabhängig voneinander für Alkyl, insbesondere C₁₋₆-Alkyl, oder Aryl, insbesondere für Phenyl oder Pyridyl stehen kann, welches unsubstituiert oder ggf. mit 1, 2, 3, 4 oder 5 Substituenten unabhängig voneinander ausgewählt aus der Gruppe bestehend aus F, Cl, Br, CF₃, C₁₋₆-Alkyl, C₁₋₆-Alkoxy, NH₂, C₁₋₆-Alkyl amino und Di(C₁₋₆-Alkyl)amino substituiert sein kann.

Beispielsweise können die erfindungsgemäß zum Einsatz kommenden funktionalisierten Polyvinylverbindungen ein Molekulargewicht (MW) von 250 bis 2,000,000, von 300 bis 2,000,000, von 350 bis 2,000,000, von 400 bis 2,000,000, von 450 bis 2,000,000, von 500 bis 2,000,000, von 550 bis 2,000,000, von 600 bis 2,000,000, von 650 bis 2,000,000, von 700 bis 2,000,000, von 750 bis 2,000,000, von 800 bis 2,000,000, von 850 bis 2,000,000, von 900 bis 2,000,000, von 950 bis 2,000,000, von 1000 bis 2,000,000, von 1050 bis 2,000,000, von 1100 bis 2,000,000, von 1150 bis 2,000,000, sowie von 1200 bis 2,000,000 Dalton.

Als funktionalisierte Polyvinylverbindung kommt bevorzugt Polyvinylalkohol oder Polyvinylamin oder deren Mischung in Betracht. Besonders bevorzugt ist die funktionalisierte Polyvinylverbindung ein Polyvinylamin.

Das Polyvinylamin und der Polyvinylalkohol können jeweils bevorzugt ein Molekulargewicht (MG) von 500 bis 1,000,000, von 550 bis 1,000,000, von 600 bis 1,000,000, von 650 bis 1,000,000, von 700 bis 1,000,000, von 750 bis 1,000,000, von 800 bis 1,000,000, von 850 bis 1,000,000, von 900 bis 1,000,000, von 950 bis 1,000,000, von 1000 bis 1,000,000, von 1050 bis 1,000,000, von 1100 bis 1,000,000, von 1150 bis 1,000,000, sowie von 1200 bis 1,000,000 Dalton oder im Bereich von 500 bis 10,000, von 550 bis 10,000, von 600 bis 10,000, von 650 bis 10,000, von 700 bis 10,000, von 750 bis 10,000, von 800 bis 10,000, von 850 bis 10,000, von 900 bis 10,000, von 950 bis 10,000, von 1000 bis 10,000, von 1050 bis 10,000, von 1100 bis 10,000, von 1150 bis 10,000, sowie von 1200 bis 10,000 Dalton aufweisen.

Geeignete Polyvinylamine umfassen insbesondere lineare Polymere und Copolymere, die sich von Vinylformamid-Monomeren ableiten und kationische und anionische Polyvinylamin-Copolymere sowie geladene und protonierte Polyvinylamine umfassen können.

Geeignete Polyvinylamine sind käuflich am Markt erhältlich, beispielsweise solche der Lupamin® Produktfamilie und hier insbesondere die Produkte Lupamin® 1595, Lupamin® 4500, Lupamin® 5095, Lupamin® 9030, Lupamin® 9050 und Lupamin® 9095. Beispiele kationischer und anionischer Polyvinylamin-Copolymere sind solche der Luredur® Produktfamilie und hier insbesondere die Produkte Luredur® Am na, Luredur® AV, Luredur® VH, Luredur® VI, Luredur® VM, Luredur® PR8094, Luredur® PR8261, und Luredur® PR8349. Beispiele geladener oder protonierter Polyvinylamine sind Produkte der Catiofast® Produktreihe und hier insbesondere die Produkte Catiofast® GM, Catiofast® PL, Catiofast® PR8236, Catiofast® VCB, Catiofast® VFH, Catiofast® VLW, Catiofast® VMP und Catiofast® VSH. Die Lupamin®, Luredur®, und Catiofast® Produkte sind erhältlich von der BASF (Florham Park, NJ, USA).

Der Fachmann versteht, dass die Kombination gemäß (ii) insbesondere so zu verstehen ist, dass es sich um die Kombination aus wenigstens einem Amino-Gruppen enthaltenden Polymer oder Oligomer als eine erste Komponente der Kombination und wenigstens einer funktionalisierten Polyvinylverbindung als eine zweite Komponente der Kombination handelt.

Als erfindungsgemäße Komponente (iii) kommen lineare oder verzweigte aliphatische C₂-C₈ Dialdehyde zum Einsatz. Vorzugsweise kommt als Additiv-Komponente (iii) Ethandial oder Glutaraldehyd, besonders bevorzugt Glutaraldehyd in Betracht.

Sofern nicht anders angegeben, beziehen sich die im Zusammenhang mit der partikelförmigen Zusammensetzung angegebenen Gewichtsangaben (Gew.-%) jeweils immer auf das Gesamtgewicht der partikelförmigen Zusammensetzung. Der Fachmann erkennt, dass die angegebenen Komponenten und Gewichtsangaben nicht für jede beliebig kleine Teilmenge der Partikel erfüllt sein müssen, sondern im Mittel über eine repräsentative Menge der hergestellten Partikel.

Die erfindungsgemäße partikelförmige Zusammensetzung kann neben den genannten Bestandteilen weitere Bestandteile enthalten. Die Art der Bestandteile sowie deren Menge hängen beispielsweise von der eingesetzten Komponente (i) ab. So kann die erfindungsgemäße partikelförmige Zusammensetzung Wasser enthalten, z.B. in einer Menge von 0,05 bis 0,5 Gew.-%, insbesondere 0,1 bis 0,3 Gew.-% und Nebenprodukte der Harnstoffsynthese wie z.B. Biuret oder NH₃. Üblicherweise beträgt der Anteil der Nebenprodukte nicht mehr als 1,5 Gew.-%, insbesondere nicht mehr als 1,25 Gew.-%.

In einer bevorzugten Ausführungsform enthält die partikelförmige Zusammensetzung als Komponente (vii) des Additivs wenigstens eine Verbindung ausgewählt aus der Gruppe der aliphatischen Dicarbonsäuren, ihren Salzen und Anhydriden, der aliphatischen Tricarbonsäuren, ihren Salzen und Anhydriden, der aromatischen Dicarbonsäuren, ihren Salzen und Anhydriden, sowie der Aldehydsäuren, ihren Salzen und Anhydriden wobei vorzugsweise der Gewichtsanteil der Komponente (i) > 60 Gew.-% und der Gewichtsanteil der Summe der Komponenten (ii), (iii) und (vii) in der Zusammensetzung < 1 Gew.-% beträgt.

Der Zusatzstoff kann entweder nur Schwefel oder nur Ammonsulfat oder wie offenbart nur mindestens ein Spurenelement umfassen. Ebenso kann der Zusatzstoff alle möglichen Kombinationen der genannten Komponenten umfassen. Der Schwefel kann sowohl elementar oder auch als Bestandteil einer Verbindung eingesetzt werden. Beispielsweise kann der Zusatzstoff Schwefel in Form von Sulfaten umfassen. Als Spurenelemente werden bevorzugt solche Elemente bezeichnet, welche für ein Lebewesen nötig sind und üblicherweise in Massenanteilen von weniger als 50 mg/kg in einem Organismus vorkommen. Spurenelemente können beispielsweise Bor, Chlor, Eisen, Kupfer, Mangan, Molybdän, und/oder Zink umfassen. Ein Fachmann erkennt, dass der Begriff Spurenelement sowohl ein einzelnes Element als auch jede mögliche Mischung von zwei oder mehreren Elementen umfasst.

In einer bevorzugten Ausführungsform sind die Komponenten der partikelförmigen Zusammensetzung homogen vermischt. Um den in der partikelförmigen Zusammensetzung enthaltenen Harnstoff ggf. mit Schwefel zu mischen, sind ggf. zusätzliche Hilfsmittel erforderlich, um eine stabile Emulsion zu bilden. Geeignete Verfahren zur Emulsionsbildung von Schwefel und Harnstoff sind einem Fachmann bekannt. Auch die zum Mischen von Harnstoff und Ammonsulfat ggf. erforderlichen Verfahrensschritte sind einem Fachmann bekannt.

Der Fachmann erkennt, dass die eingesetzten Komponenten (ii), (iii), (iv), (v), (vi) und (vii) bei der Herstellung der partikelförmigen Zusammensetzung ggf. untereinander und ggf. auch mit der Harnstoff-Komponente (i) teilweise oder sogar vollständig in Wechselwirkung treten können. So ist beispielsweise die Vernetzung unter Ausbildung von kovalenten Bindungen für Aldehyde bzw. Carbonsäureanhydride mit Harnstoff bekannt oder die Bildung von Komplexen aus Harnstoff und Carbonsäuren. Komponenten, wie z.B. Polyvinylalkohol und Polyvinylamin neigen zur Ausbildung von Wasserstoffbrückenbindungen. Ggf. liegen die zur Herstellung der partikelförmigen Zusammensetzung eingesetzten Komponenten in dem erhaltenen Endprodukt daher in teilweise oder vollständig modifizierter Form vor. Erfindungsgemäß sind auch derartige modifizierte Komponenten mitumfasst.

Die erfindungsgemäße partikelförmige Zusammensetzung enthält
(i) Harnstoff;
und ein Additiv umfassend die Komponente (ii) und eine oder beide der Komponenten (iii) und (vii):
(ii) Kombination aus Polyethylenimin und Polyvinylalkohol oder Kombination aus Polyethylenimin und Polyvinylamin;
(iii) wenigstens einen aliphatischen C₂₋C₈ Dialdehyd;
(vii) wenigstens eine Verbindung ausgewählt aus der Gruppe der aliphatischen Dicarbonsäuren, ihren Salzen und Anhydriden, der aliphatischen Tricarbonsäuren, ihren Salzen und Anhydriden, der aromatischen Dicarbonsäuren, ihren Salzen und Anhydriden, sowie der Aldehydsäuren, ihren Salzen und Anhydriden;
und einen Zusatzstoff umfassend eine oder mehrere der Komponenten (iv) bis (v):
(iv) Schwefel;
(v) Ammonsulfat;
wobei vorzugsweise der Gewichtsanteil der Komponente (i) > 50 Gew.-% und der Gewichtsanteil der Summe der Komponenten (ii), (iii) und (vii) in der Zusammensetzung < 1 Gew.-% beträgt und der Gewichtsanteil der Summe der Komponenten (iv) und (v) in der Zusammensetzung < 50 Gew.-% beträgt.

Sofern die erfindungsgemäße Zusammensetzung eine aliphatische Dicarbonsäure als Komponente (vii) aufweist, kann diese bevorzugt ausgewählt werden aus der Gruppe bestehend aus Oxalsäure, Malonsäure, Bernsteinsäure, Glutarsäure, Adipinsäure, Pimelinsäure, Suberinsäure, Azealinsäure, Sebacinsäure, Undecandisäure, Dodecandisäure, Tridecandisäure, Tetradecandisäure, Hexadecandisäure, sowie jeweils deren Salzen und Anhydriden. Besonders bevorzugt liegt als Dicarbonsäure der Komponente (vii) Oxalsäure, Bernsteinsäure oder eine Mischung dieser beiden Säuren vor. Sofern die erfindungsgemäße Zusammensetzung eine aliphatische Tricarbonsäure als Komponente (vii) aufweist, kann diese bevorzugt ausgewählt werden aus der Gruppe bestehend aus Zitronensäure, Iso-Zitronensäure, sowie jeweils deren Salzen und Anhydriden. Besonders bevorzugt liegt als Tricarbonsäure der Komponente (vii) Zitronensäure vor.

Weist die erfindungsgemäße Zusammensetzung als Komponente (vii) eine aromatische Dicarbonsäure oder deren Anhydrid auf, so kann diese bevorzugt ausgewählt werden aus der Gruppe bestehend Phthalsäure, Phthalsäureanhydrid, Isophthalsäure und Terephthalsäure. Besonders bevorzugt liegt als aromatische Dicarbonsäure der Komponente (vii) bzw. deren Anhydrid Phthalsäure, Phthalsäureanhydrid oder deren Mischung vor.

Sofern die erfindungsgemäße Zusammensetzung eine Aldehydsäure als Komponente (vii) aufweist, ist diese bevorzugt Glyoxylsäure.

Sofern eine Säuren der Komponente (vii) in Form ihres Salzes vor, so kommen insbesondere Salze der Alkalimetalle wie beispielsweise Natrium und Kalium und der Erdalkalimetalle wie beispielsweise Calcium und Magnesium in Betracht, aber auch Ammoniumsalze, insbesondere vom Typ [NHₓR₄₋ₓ]⁺, worin x = 0, 1, 2, 3 oder 4 und R für einen linearen oder verzweigten C₁₋₄ Alkyl-Rest steht.

In einer weiteren bevorzugten Ausführungsform enthält die partikelförmige Zusammensetzung
(i) Harnstoff;
und ein Additiv umfassend die Komponente (ii) und eine oder beide der Komponenten (iii) und (vii):
(ii) Kombination aus Polyethylenimin und Polyvinylamin;
(iii) Ethandial und/oder Glutaraldehyd;
(vii) wenigstens eine Verbindung ausgewählt aus der Gruppe bestehend aus Oxalsäure, Bernsteinsäure, Zitronensäure, Phthalsäure, Phthalsäureanhydrid, Glyoxylsäure und deren Salzen,
und einen Zusatzstoff umfassend eine oder mehrere der Komponenten (iv) bis (v):
(iv) Schwefel;
(v) Ammonsulfat;
wobei vorzugsweise der Gewichtsanteil der Komponente (i) > 50 Gew.-% und der Gewichtsanteil der Summe der Komponenten (ii), (iii) und (vii) in der Zusammensetzung < 1 Gew.-% beträgt und der Gewichtsanteil der Summe der Komponenten (iv) und (v) in der Zusammensetzung < 50 Gew.-% beträgt.

Ganz besonders bevorzugte Ausführungsformen der partikelförmigen Zusammensetzung enthalten
(i) Harnstoff;
und ein Additiv ausgewählt aus der Gruppe (a)-(i)
(a) Additiv umfassend (ii) eine Kombination aus Polyethylenimin und Polyvinylamin;
(b) Additiv umfassend (iii) Glutaraldehyd;
(c) Additiv umfassend (ii) eine Kombination aus Polyethylenimin und Polyvinylamin und (vii) Oxalsäure;
(d) Additiv umfassend (ii) eine Kombination aus Polyethylenimin und Polyvinylamin und (vii) Zitronensäure;
(e) Additiv umfassend (ii) eine Kombination aus Polyethylenimin und Polyvinylamin und (vii) Bernsteinsäure;
(f) Additiv umfassend (ii) eine Kombination aus Polyethylenimin und Polyvinylamin und (vii) Phthalsäure;
(g) Additiv umfassend (ii) eine Kombination aus Polyethylenimin und Polyvinylamin und (vii) Phthalsäureanhydrid;
(h) Additiv umfassend (ii) eine Kombination aus Polyethylenimin und Polyvinylamin und (vii) Glutaraldehyd;
(i) Additiv umfassend (ii) eine Kombination aus Polyethylenimin und Polyvinylamin und (vii) Glyoxylsäure;
und einen Zusatzstoff umfassend eine oder mehrere der Komponenten (iv) bis (vi):
(iv) Schwefel;
(v) Ammonsulfat;
;
wobei vorzugsweise der Gewichtsanteil der Komponente (i) > 50 Gew.-% und der Gewichtsanteil der Summe der Komponenten (ii), (iii) und (vii) in der Zusammensetzung < 1 Gew.-% beträgt und der Gewichtsanteil der Summe der Komponenten (iv) und (v) in der Zusammensetzung < 50 Gew.-% beträgt.

Der Gewichtsanteil der Komponte (i) in der partikelförmigen Zusammensetzung beträgt vorzugsweise > 50 Gew.-%, besonders bevorzugt > 60 Gew.-%, ganz besonders bevorzugt > 96 Gew.-%.

Der Gewichtsanteil der Additiv-Komponente kann variieren, beispielsweise in Abhängigkeit von den eingesetzten Komponenten (ii), (iii) und (vii). Vorzugsweise beträgt der Gewichtsanteil der Summe der Komponenten (ii), (iii) und (vii) in der partikelförmigen Zusammensetzung < 0,5 Gew.-%, besonders bevorzugt < 0,4 Gew.-%, ganz besonders bevorzugt < 0,3 Gew.-% und noch weiter bevorzugt < 0,25 Gew.-%.

Sofern die Additiv-Komponente zwei oder mehr Komponenten umfasst, können auch deren relative Anteile variieren. So kann beispielsweise das Gewichtsverhältnis der Komponenten (ii) und (iii) oder das Gewichtsverhältnis der Komponenten (ii) und (vii) im Bereich von 1:20 bis 20:1, vorzugsweise von 1:15 bis 15:1, besonders bevorzugt 1:10 bis 10:1 liegen und inkrementelle Werte dazwischen umfassen.

Ganz besonders bevorzugte Ausführungsformen der partikelförmigen Zusammensetzung enthalten eine Kombination aus Polyethylenimin und Polyvinylamin. Das Gewichtsverhältnis von Polyethlyenimin und Polyvinylamin innerhalb der Kombination dieser beiden Komponenten kann variieren, beispielsweise im Bereich von 1:20 bis 20:1, vorzugsweise von 1:15 bis 15:1, besonders bevorzugt 1:10 bis 10:1 und inkrementelle Werte dazwischen umfassen.

Des Weiteren kann auch das Gewichtsverhältnis der Kombination aus den beiden Komponenten Polyethlyenimin und Polyvinylamin zu Komponente (iii) oder das Gewichtsverhältnis der Kombination aus den beiden Komponenten Polyethlyenimin und Polyvinylamin zu Komponente (vii) variieren und jeweils beispielsweise im Bereich von 1:20 bis 20:1, vorzugsweise von 1:15 bis 15:1, besonders bevorzugt 1:10 bis 10:1 und inkrementelle Werte dazwischen umfassen.

Sofern der Zusatzstoff zwei oder mehr Komponenten umfasst, können auch deren relative Anteile variieren. So kann beispielsweise das Gewichtsverhältnis der Komponenten (iv) und (v) oder das Gewichtsverhältnis der Komponenten (iv) und (vi) und oder das Gewichtsverhältnis der Komponenten (v) und (vi) im Bereich von 1:20 bis 20:1, vorzugsweise von 1:15 bis 15:1, besonders bevorzugt 1:10 bis 10:1 liegen und inkrementelle Werte dazwischen umfassen.

In einer bevorzugten Ausführungsform ist die erfindungsgemäße partikelförmige Zusammensetzung im Wesentlichen frei von Formaldehyd. Der Ausdruck "im Wesentlichen frei von Formaldehyd" bedeutet im Sinne der vorliegenden Erfindung, dass die Zusammensetzung < 0,1 Gew.-%, vorzugsweise < 0,05 Gew.-%, besonders bevorzugt < 0,005 Gew.-% und noch weiter bevorzugt < 0,0005 Gew.-% Formaldehyd aufweist.

Ein weiterer Aspekt der vorliegenden Erfindung betrifft die Verwendung eines Additivs und/oder eines Zusatzstoffs wie vorstehend beschrieben zur Herstellung einer partikelförmigen Zusammensetzung enthaltend Harnstoff.

Alle bevorzugten Ausführungsformen, welche vorstehend im Zusammenhang mit der erfindungsgemäßen partikelförmigen Zusammensetzung beschrieben wurden, gelten entsprechend auch für die erfindungsgemäße Verwendung des Additivs bzw. des Zusatzstoffs zur Herstellung einer partikelförmigen Zusammensetzung enthaltend Harnstoff und werden daher an dieser Stelle nicht wiederholt.

Ein weiterer Aspekt der Erfindung betrifft ein Verfahren zur Herstellung einer partikelförmigen Zusammensetzung enthaltend Harnstoff umfassend die Schritte
(A) Bereitstellen einer Harnstoff-haltigen Lösung;
(B) Granulieren der Harnstoff-haltigen Lösung in einem Fließbett, in einer Granuliertrommel oder auf einem Granulierteller oder Prillieren der Harnstoff-haltigen Lösung, jeweils unter Zugabe eines Additivs und/oder eines Zusatzstoffs mit einer Zusammensetzung wie vorstehend beschrieben.

Alle bevorzugten Ausführungsformen, welche vorstehend im Zusammenhang mit der erfindungsgemäßen partikelförmigen Zusammensetzung beschrieben wurden, gelten entsprechend auch für das erfindungsgemäße Verfahren zur Herstellung einer partikelförmigen Zusammensetzung enthaltend Harnstoff und werden daher an dieser Stelle nicht wiederholt.

In einer bevorzugten Ausführungsform des erfindungsgemäßen Verfahrens beträgt der Harnstoff-Gehalt der in Schritt (A) eingesetzten Lösung > 50 Gew.-%, vorzugsweise > 60 Gew.-%, besonders bevorzugt > 70 Gew.-%, ganz besonders bevorzugt > 80 Gew.-%, noch weiter bevorzugt > 96 Gew.-%.

Das Granulieren der Harnstoff-haltigen Lösung unter Zugabe eines Additivs und/oder eines Zusatzstoffs gemäß Schritt (B) kann nach üblichen, dem Fachmann bekannten Methoden erfolgen, beispielsweise mittels Sprühkristallisation (Prilling), Trommelgranulierung oder Fließbettgranulierung.

In einer bevorzugten Ausführungsform des erfindungsgemäßen Verfahrens erfolgt die Granulierung in Schritt (B) mittels Granulierung in einem Fließbett, in einer Granuliertrommel oder auf einem Granulierteller oder mittels Prillierung. Bevorzugt wird die Harnstoff-haltige Lösung in Schritt (B) mittels Fließbettgranulierung granuliert, umfassend die Schritte:
(B1) Bereitstellen von Harnstoff-haltigen Keimen;
(B2) Fluidisieren der Harnstoff-haltigen Keime
(B3) Aufsprühen der Harnstoff-haltigen Lösung unter Verwendung eines Additivs und/oder eines Zusatzstoffs mit einer Zusammensetzung wie vorstehend beschrieben.

Sofern das Additiv und/oder der Zusatzstoff zwei oder mehr Komponenten umfasst, können diese jeweils einzeln oder zusammen, oder auch in Form von Vormischungen in dem erfindungsgemäßen Verfahren eingesetzt werden. Die Zeitpunkte und Zugabe der Komponenten können variieren. So ist es beispielsweise möglich, eine oder mehrere der Komponenten in die bereitgestellte Harnstofflösung zu geben oder auch eine oder mehrere der Komponenten erst unmittelbar vor dem Aufsprühen der Harnstoff-haltigen Lösung dieser zuzugeben. In Abhängigkeit von der Beschaffenheit der Komponenten kann es vorteilhaft sein, die Komponenten in Form von Lösungen, Suspensionen, Emulsionen oder ähnlichem einzusetzen. Als geeignete Flüssigkeiten für die Lösungen oder sonstige Formulierungen kommen insbesondere Wasser, aber auch organische Lösungsmittel wie beispielsweise Alkohole, Ether, usw. in Betracht.

Die Temperatur der Harnstoff-haltigen Lösung beträgt vorzugsweise > 120°C, besonders bevorzugt 125°C und ganz besonders bevorzugt > 130°C.

In einer Ausführungsform des erfindungsgemäßen umfasst das Verfahren den Schritt (C):
(C): Auftrennung der partikelförmige Harnstoff-Zusammensetzung nach ihrer Herstellung in drei Fraktionen aufgetrennt, wobei
eine Fraktion (F1) Partikel mit der gewünschten Zielgröße enthält,
eine Fraktion (F2) Partikel mit einer Größe oberhalb der gewünschten Zielgröße enthält, und
eine Fraktion (F3) Partikel mit einer Größe unterhalb der gewünschten Zielgröße enthält, und
wobei vorzugsweise die Fraktion F2 nach einer Zerkleinerung der Partikel und die Fraktion F3 wieder in das Verfahren zurückgeführt wird.

In Anlagen zur Herstellung von Harnstoff und dessen Weiterverarbeitung zu partikelförmigen Zusammensetzungen fällt üblicherweise auch Ammoniak an. Dieser kann durch eine Wäsche mit geeigneten Säuren, wie beispielsweise Salpetersäure oder Schwefelsäure in die entsprechenden Ammoniumsalze, wie beispielsweise Ammoniumnitrat oder Ammoniumsulfat überführt und diese einer weiteren Verwendung, beispielsweise in Düngemitteln zugeführt werden. Geeignete Verfahren und Durchführung der Säurewäsche sind beispielsweise in der WO2010/060535 beschrieben.

In einer weiteren Ausführungsform umfasst das erfindungsgemäße Verfahren den Schritt (D):
(D) Säurewäsche.

Die Säurewäsche kann vorteilhafterweise auch unter Einsatz der vorstehend beschriebenen Säuren der Komponente (iv) erfolgen, so dass die so erhaltenen Salze dann als Komponente (iv) in dem erfindungsgemäß verwendeten Additiv genutzt werden können.

Eine weiterere Offenbarung der vorliegenden Erfindung betrifft eine partikelförmige Zusammensetzung erhältlich nach einem erfindungsgemäßen Verfahren wie vorstehend beschrieben.

Ein weiterer Aspekt der vorliegenden Erfindung ist die Verwendung einer partikelförmigen Harnstoff-Zusammensetzung wie vorstehend beschrieben als Düngemittel.

Eine weiterere Offenbarung der Erfindung betrifft eine Vorrichtung zur Herstellung einer partikelförmigen Zusammensetzung enthaltend Harnstoff umfassend:
(a) einen Granulator;
(b) wenigstens ein Mittel für die Zugabe eines Additivs und/oder eines Zusatzstoffs wie vorstehend beschrieben;
(c) wenigstens ein Mittel für die Auftrennung der partikelförmigen Zusammensetzung in Fraktionen unterschiedlicher Partikelgröße;
(d) ggf. wenigstens ein Mittel für die Durchführung einer Säurewäsche.

In einer bevorzugten Ausführungsform der erfindungsgemäßen Vorrichtung ist der Granulator (a) ein Fließbettgranulator.

Die offenbarte Vorrichtung eignet sich besonders zur Durchführung des erfindungsgemäßen Verfahrens. Eine weitere Offenbarung der Erfindung betrifft daher die Verwendung der erfindungsgemäßen Vorrichtung zur Durchführung des erfindungsgemäßen Verfahrens zur Herstellung einer partikelförmigen Zusammensetzung enthaltend Harnstoff.

Die erfindungsgemäßen Verfahren und die offenbarte Vorrichtung können beispielsweise mit einer Anlage zur Herstellung von Harnstoff kombiniert werden.

Im Folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1:

In einer Versuchsanlage wurde Harnstoff mit 7 Gew.-% elementaren Schwefel in einem Fließbettgranulator mit einem zylindrischen Fließbett von 40 cm Durchmesser bei einer Temperatur von circa 108°C granuliert. Das Fließbett war auf der Unterseite mit einer perforierten Platte abgeschlossen, deren Löcher einen Durchmesser von 2,0 mm aufwiesen. Die Fluidisierungsluft strömte mit einer oberflächlichen Geschwindigkeit von circa 2 m/s in das Fließbett. Ein Überlauf wurde 10 cm oberhalb der Bodenplatte an der Seitenwand des Bettes angebracht. Eine definierte Menge (circa 45 kg) an Partikeln oder Granulat mit einer engen Größenverteilung wurde dann als Keime für die Granulierung in die Säule des Granulators gegeben. Das Bett mit den Keimen (circa 50 cm tief) wurde mit heißer Luft bei einer Temperatur von circa 100°C fluidisiert und mit der Zugabe von 96 bis 97 Gew.-%-iger Harnstofflösung mit einer Temperatur von circa 135°C begonnen, sobald das Bett die für den Lauf vorgesehene Temperatur von ca. 108°C erreicht hatte. Aus einem Vorratstank wurde dann die Lösung bestehend aus Harnstoff und Schwefel mit einem Wassergehalt von 3-4 Gew-% mit einer Rate von 350 kg/h über eine Spritzdüse, welche bei einer Temperatur von circa 140°C mit Luft, zugeführt mit einer Rate von 240 kg/h, betrieben wurde, in den Fließbettgranulator gegeben. Die gemäß der nachstehenden Tabelle 1 eingesetzten Additive für die Granulierung wurden dann bei circa 135°C mit der Lösung bestehend aus Harnstoff und Schwefel vermischt. Feststoffe wurden in regelmäßigen Intervallen von 5 Minuten über einen Auslass aus dem Fließbett abgeführt, um eine weitgehend konstante Höhe des Bettes zu erreichen. Die Proben der so entnommenen Feststoffe wurden dann jeweils gesiebt, um deren Größenverteilung zu bestimmen. Es wurden keine Feststoffe in den Fließbettgranulator zurückgeführt. Die Dauer pro Charge betrug jeweils circa 30 Minuten. Nach Ablauf dieser Zeit wurde die Zufuhr unterbrochen, das Granulat auf circa 100°C abgekühlt, aus dem Fließbettgranulator entfernt und zu seiner Auftrennung in die unterschiedlichen Fraktionen gesiebt. Die Fraktion mit der gewünschten Größenverteilung wurde dann auf circa 60°C abgekühlt, um deren Produkteigenschaften zu analysieren. Alle Fraktionen wurden gewogen, um die Wachstumsrate des Granulates zu ermitteln. Des Weiteren wurde auch der Staub aus den Taschenfiltern der Abluftvorrichtung gesammelt und gewogen.

Entsprechend der vorstehend beschriebenen Vorgehensweise wurden auch Vergleichsversuche zur Granulierung ohne Additivzusatz sowie mit Polyvinylamin (PVA), einer Polyvinylamin/Polyethylenimin-Mischung oder einem Standardadditiv (Harnstoff-Formaldehyd Additiv UF80) durchgeführt und das jeweils erhaltene Granulat entsprechend aufgearbeitet und analysiert.

Die nachstehende Tabelle 1 gibt die entsprechende Beurteilung der Granulate in Hinblick auf die Staubbildung, Druckfestigkeit, Dichte und Verklumpung wieder. Die ebenfalls angegebene Empfindlichkeit für die Staubbildung ist das Ergebnis einer visuellen Beurteilung von aufgefangenem Staub aus einem kleinen Fließbettkühler. Die verwendete Skala für die Bewertung der erhaltenen Granulate ist in Tabelle 1b wiedergegeben.

Geeignete Analysenmethoden für die Produkteigenschaften sind dem Fachmann bekannt. Beispielhaft sei auf die ISO 3944 oder IFDC S-112 zur Bestimmung der Schüttdichte, die IFDC S-116 zur Bestimmung der Staubbildung, die IFDC S-115 zur Bestimmung der Druckfestigkeit sowie die IFDC S-106 zur Bestimmung der Verklumpung verwiesen, wobei es sich bei den IFDC-Methoden um solche des International Fertilizer Development Center, Muscle Shoals, Alabama, USA handelt.

**Tabelle 1:**

| Additiv Gew % | | --- | UF80 | PVA/PEI 88/12 | PVA/PEI/PVAI 44/6/50 | PVA/PEI/CA 34,5/3,5/62 | PVA / PEI / PVAI / CA 17,2/1,8/50/31 | PVA/PEI/CA 80/10/10 |
|---|---|---|---|---|---|---|---|---|
| Erfindungsgemäß (E) / Vergleich (V) | | V | V | E | E | E | E | E |
| Dosierung mg/kg | | 0 | 3000 | 1000 | 2000 | 2000 | 2000 | 1000 |
| Parameter | gut bedeutet | | | | | | | |
| Staub im Granulatorfilter | niedrig | 5 | 2 | 2 | 2 | 1 | 2 | 2 |
| Staubbildung Abkühlung | niedrig | 5 | 2 | 2 | 2 | 1 | 2 | 2 |
| Verklumpung % | keine | 3 | 1 | 1 | 1 | 1 | 1 | 1 |
| Klumpenhärte | FF* | 3 | 1 | 1 | 1 | 1 | 1 | 1 |
| Druckfestigkeit | Hoch | 4 | 2 | 2 | 2 | 1 | 2 | 2 |
| Schüttdichte (lose) | hoch | 3 | 1 | 1 | 2 | 1 | 1 | 1 |
| | | | | | | | | |
| Beurteilung (nicht gewichtet) | | 23 | 9 | 9 | 10 | 6 | 9 | 9 |

| | | | | | | | | |
|---|---|---|---|---|---|---|---|---|
| PVA: Polyvinylamin PEI: Polyethylenimin PVAI Polyvinylalkohol CA Zitronensäure FF frei fließend | | | | | | | | |

**Tabelle 2:**

| Skala | Staub im Filter (%) | Staub Abkühlen | Druckfestigkeit kg | Schüttdichte (g/l) | Klumpen (%) | Härte (kg) |
|---|---|---|---|---|---|---|
| 1 | 0-4 | 0 | >3,5 | >675 | 0 | keine |
| 2 | >4-6 | 1 | >3,0-3,5 | 675-665 | 0-10 | gering |
| 3 | >6-8 | 2 | >2,5-3,0 | <665-655 | 11-20 | mittel |
| 4 | >8-10 | 2-3 | >2,0-2,5 | <655-645 | 21-30 | hart |
| 5 | >10 | 3 | <2,0 | <645 | >30 | |

### Beispiel 2:

Entsprechend der in Beispiel 1 beschriebenen Vorgehensweise wurde der Einfluss eines erfindungsgemäßen Granulierungsadditivs aus Zitronensäure in verschiedenen Dosierungen und Mischungen aus Polyethyleniminin und Polyvinylamin bestimmt. Hierbei wurde die Zitronensäure gemischt mit Polyethyleniminin / Polyvinylamin und dosiert dem der Düse zugeführten Strom aus Harnstoff und Schwefel vor dem Sprühen zugeführt. Die so erhaltene Lösung bestehend aus Harnstoff und Schwefel mit einem Wassergehalt von 3 Gew.-% wurde dann bei einer Temperatur von 132 °C mit einer Rate von 350 kg/h zugeführt und die Aufarbeitung erfolgte wie in Beispiel 1 beschrieben. Ein entsprechender Vergleichsversuch mit Formaldehyd wurde ebenfalls durchgeführt.

In Tabelle 1 ist jeweils der Anteil an Staub im Fließbettgranulator wiedergegeben und gibt auch die entsprechende Beurteilung der Granulate von Beispiel 2

### Beispiel 3:

Entsprechend der in Beispiel 1 beschriebenen Vorgehensweise wurde der Einfluss erfindungsgemäßer Granulierungsadditive bestehend aus einer Mischung aus 500 mg/kg Polyethyleniminin und Polyvinylamin (40 Gew.-%/60 Gew.-%, jeweils bezogen auf die Mischung aus Polyethylenimin und Polyvinylamin) mit Oxalsäure, Zitronensäure, Bernsteinsäure, Phthalsäure, Phthalsäureanhydrid, Glutaraldehyd sowie Glyoxylsäure auf die Granulierung von Harnstoff und Ammoniumsulfat bestimmt. Oxalsäure, Zitronensäure, Bernsteinsäure, Phthalsäure, Phthalsäureanhydrid und Glutaraldehyd wurden jeweils in den Harnstoffvorrat gegeben, die Glyoxylsäure sowie die Mischung aus Polyethyleniminin und Polyvinylamin wurden jeweils vor dem Sprühen der der Düse zugeführten Lösung bestehend aus Harnstoff und Ammonsulfat zugegeben. Auch hier wurde die so erhaltene Lösung mit einem Wassergehalt von 3 Gew.-% dann bei einer Temperatur von 132 °C mit einer Rate von 350 kg/h zugeführt und die Aufarbeitung erfolgte wie in Beispiel 1 beschrieben. Ein entsprechender Vergleichsversuch mit Formaldehyd wurde ebenfalls durchgeführt.

| | Erfindungsgemäß (E) / Vergleich (V) | Dosierung (mg/kg) | Druckfestigkeit (in kg) | Staubbildung (in %) |
|---|---|---|---|---|
| Kein Additiv | V | 0 | 2,26 | 10,85 |
| Formaldehyd | V | 4500 | 3,75 | 3,90 |
| Oxalsäure | E | 1000 | 4,48 | 2,81 |
| Zitronensäure | E | 1000 | 4,05 | 4,44 |
| Bernsteinsäure | E | 1000 | 3,63 | 3,70 |
| Phthalsäure | E | 1000 | 3,84 | 3,70 |
| Phthalsäureanhydrid | E | 1000 | 4,72 | 2,48 |
| Glutaraldehyd | E | 1000 | 3,71 | 3,65 |
| Glyoxylsäure | E | 1500 | 5,07 | 2,74 |

Die Untersuchungen der gemäß den Beispielen 1-3 erhaltenen Granulate hat ergeben, dass sowohl die Staubbildung als auch die Eigenschaften des Granulats (Druckfestigkeit, Neigung zur Verklumpung) sich bei Zugabe der erfindungsgemäßen Additive verbesserten. Das Ergebnis war mit den bei der Verwendung von Formaldehyd erhaltenen Ergebnissen vergleichbar oder sogar besser, wobei wesentlich geringere Mengen an Additiv benötigt wurden.

## Patentansprüche

1. Partikelförmige Zusammensetzung enthaltend
(i) Harnstoff;
und ein Additiv umfassend die Komponente (ii) und eine oder beide der Komponenten (iii) und (vii):
(ii) Kombination aus Polyethylenimin und Polyvinylalkohol oder Kombination aus Polyethylenimin und Polyvinylamin;
(iii) wenigstens einen aliphatischen C₂₋C₈ Dialdehyd;
(vii) wenigstens eine Verbindung ausgewählt aus der Gruppe der aliphatischen Dicarbonsäuren, ihren Salzen und Anhydriden, der aliphatischen Tricarbonsäuren, ihren Salzen und Anhydriden, der aromatischen Dicarbonsäuren, ihren Salzen und Anhydriden, sowie der Aldehydsäuren, ihren Salzen und Anhydriden;
und einen Zusatzstoff umfassend eine oder mehrere der Komponenten (iv) bis (v):
(iv) Schwefel;
(v) Ammonsulfat;
wobei der Gewichtsanteil der Komponente (i) > 50 Gew.-% und der Gewichtsanteil der Summe der Komponenten (ii), (iii) und (vii) in der Zusammensetzung < 1 Gew.-% beträgt und der Gewichtsanteil der Summe der Komponenten (iv) und (v) in der Zusammensetzung < 50 Gew.-% beträgt.

2. Die partikelförmige Zusammensetzung gemäß Anspruch 1 enthaltend
(i) Harnstoff;
und ein Additiv umfassend die Komponente (ii) und eine oder beide der Komponenten (iii) und (vii):
(ii) Kombination aus Polyethylenimin und Polyvinylamin;
(iii) Ethandial und/oder Glutaraldehyd;
(vii) wenigstens eine Verbindung ausgewählt aus der Gruppe bestehend aus Oxalsäure, Bernsteinsäure, Zitronensäure, Phthalsäure, Phthalsäureanhydrid, Glyoxylsäure und deren Salzen
und einen Zusatzstoff umfassend eine oder mehrere der Komponenten (iv) bis (v):
(iv) Schwefel;
(v) Ammonsulfat;
wobei vorzugsweise der Gewichtsanteil der Komponente (i) > 50 Gew.-% und der Gewichtsanteil der Summe der Komponenten (ii), (iii) und (vii) in der Zusammensetzung < 1 Gew.-% beträgt und der Gewichtsanteil der Summe der Komponenten (iv) und (v) in der Zusammensetzung < 50 Gew.-% beträgt.

3. Die partikelförmige Zusammensetzung gemäß einem der Ansprüche 1-2, wobei das Polyethylenimin der Komponente (ii) ein Molekulargewicht im Bereich von 500 - 2,000,000 Da aufweist.

4. Die partikelförmige Zusammensetzung gemäß einem der Ansprüche 1-3, wobei das Polyvinylamin der Komponente (ii) ein Molekulargewicht im Bereich von 500 - 1,000,000 Da aufweist.

5. Die partikelförmige Zusammensetzung gemäß einem der Ansprüche 1-4, **dadurch gekennzeichnet, dass** der Gewichtsanteil der Summe der Komponenten (ii), (iii) und (vii) in der Zusammensetzung < 0,5 Gew.-%, bevorzugt < 0,4 Gew.-%, besonders bevorzugt < 0,3 Gew.-% und ganz besonders bevorzugt < 0,25 Gew.-% beträgt.

6. Verwendung eines Additivs und/oder eines Zusatzstoffs wie in einem der Ansprüche 1-5 definiert zur Herstellung einer partikelförmigen Zusammensetzung enthaltend Harnstoff.

7. Ein Verfahren zur Herstellung einer partikelförmigen Zusammensetzung enthaltend Harnstoff umfassend die Schritte
(A) Bereitstellen einer Harnstoff-haltigen Lösung;
(B) Granulieren oder Prillieren der Harnstoff-haltigen Lösung unter Zugabe eines Additivs und/oder eines Zusatzstoffs definiert wie in einem der Ansprüche 1-5 definiert.

8. Das Verfahren gemäß Anspruch 7, wobei der Harnstoff-Gehalt der Lösung > 10 Gew.-%, vorzugsweise > 50 Gew.-% beträgt.

9. Das Verfahren gemäß einem der Ansprüche 7 oder 8, wobei Harnstoff-haltige Lösung in Schritt (B) mittels Fließbettgranulierung granuliert wird, umfassend die Schritte
(B1) Bereitstellen von Harnstoff-haltigen Keimen;
(B2) Fluidisieren der Harnstoff-haltigen Keime;
(B3) Aufsprühen der Harnstoff-haltigen Lösung auf die Keime unter Verwendung eines Additivs und/oder eines Zusatzstoffs wie in einem der Ansprüche 1-3 definiert.

10. Das Verfahren gemäß einem der Ansprüche 7-9, wobei die Temperatur der Harnstoff-haltigen Lösung >120°C, vorzugsweise > 125°C, besonders bevorzugt > 130°C beträgt.

11. Das Verfahren gemäß einem der Ansprüche 7-10 umfassend den Schritt (C):
(C) Auftrennung der partikelförmigen Harnstoff-Zusammensetzung nach ihrer Herstellung in drei Fraktionen aufgetrennt wird, wobei
eine Fraktion (F1) Partikel mit der gewünschten Zielgröße enthält,
eine Fraktion (F2) Partikel mit einer Größe oberhalb der gewünschten Zielgröße enthält, und
eine Fraktion (F3) Partikel mit einer Größe unterhalb der gewünschten Zielgröße enthält.

12. Das Verfahren gemäß einem der Ansprüche 7-11 umfassend den Schritt (D)
(D) Nasswäsche.

13. Verwendung einer partikelförmigen Harnstoff-Zusammensetzung nach einem der Ansprüche 1-5 als Düngemittel.

## Claims

1. Particulate composition comprising
(i) urea;
and an additive comprising component (ii) and one or both of components (iii) and (vii):
(ii) combination of polyethyleneimine and polyvinyl alcohol or combination of polyethyleneimine and polyvinylamine;
(iii) at least one aliphatic C₂-C₈ dialdehyde;
(vii) at least one compound selected from the group of the aliphatic dicarboxylic acids and the salts and anhydrides thereof, the aliphatic tricarboxylic acids and the salts and anhydrides thereof, the aromatic dicarboxylic acids and the salts and anhydrides thereof, and the aldehyde acids and the salts and anhydrides thereof;
and an additive comprising one or more of components (iv) to (v):
(iv) sulfur:
(v) ammonium sulfate;
wherein the content by weight of component (i) is > 50% by weight and the content by weight of the sum of components (ii), (iii) and (vii) in the composition is < 1% by weight and the content by weight of the sum of components (iv) and (v) in the composition is < 50% by weight.

2. The particulate composition according to Claim 1 comprising
(i) urea;
and an additive comprising component (ii) and one or both of components (iii) and (vii):
(ii) combination of polyethyleneimine and polyvinyl-amine
(iii) ethanedial and glutaraldehyde;
(vii) at least one compound selected from the group consisting of oxalic acid, succinic acid, citric acid, phthalic acid, phthalic anhydride, glyoxylic acid and the salts thereof;
and an additive comprising one or more of components (iv) to (v):
(iv) sulfur:
(v) ammonium sulfate;
wherein preferably the content by weight of component (i) is > 50% by weight and the content by weight of the sum of components (ii), (iii) and (vii) in the composition is < 1% by weight and the content by weight of the sum of components (iv) and (v) in the composition is < 50% by weight.

3. The particulate composition according to either of Claims 1-2, wherein the polyethyleneimine in component (ii) has a molecular weight in the range from 500 to 2 000 000 Da.

4. The particulate composition according to any of Claims 1-3, wherein the polyvinylamine in component (ii) has a molecular weight in the range from 500 to 1 000 000 Da.

5. The particulate composition according to any of Claims 1-4, **characterized in that** the content by weight of the sum of components (ii), (iii) and (vii) in the composition is < 0.5% by weight, preferably < 0.4% by weight, more preferably < 0.3% by weight and most preferably < 0.25% by weight.

6. Use of an additive and/or an added substance as defined in any of Claims 1-5 in the production of a particulate composition containing urea.

7. A process for producing a particulate composition containing urea, comprising the steps of
(A) providing a urea-containing solution
(B) granulating or prilling the urea-containing solution with addition of an additive and/or added substance defined as defined in any of Claims 1-5.

8. The process according to Claim 7, wherein the urea content of the solution is > 10% by weight, preferably > 50% by weight.

9. The process according to either of Claims 7 or 8, wherein the urea-containing solution in step (B) is granulated by fluidized-bed granulation comprising the steps of
(B1) providing urea-containing seeds;
(B2) fluidizing the urea-containing seeds;
(B3) spraying the urea-containing solution onto the seeds using an additive or added substance as defined in Claims 1-3.

10. The process according to any of Claims 7-9, wherein the temperature of the urea-containing solution is > 120°C, preferably > 125°C, more preferably > 130°C.

11. The process according to any of Claims 7-10, including step (C):
(C) dividing the particulate urea composition after the production thereof is divided into three fractions, wherein
one fraction (F1) comprises particles having the desired target size,
one fraction (F2) comprises particles having a size above the desired target size, and
one fraction (F3) comprises particles having a size below the desired target size.

12. The process according to any of Claims 7-11, including step (D):
(D) wet washing.

13. Use of a particulate urea composition according to any of Claims 1-5 as a fertilizer.

## Revendications

1. Composition particulaire contenant :
(i) de l'urée ;
et un additif comprenant le composant (ii) et un ou deux des composants (iii) et (vii) :
(ii) une combinaison de polyéthylène-imine et d'alcool polyvinylique ou une combinaison de polyéthylène-imine et de polyvinylamine ;
(iii) au moins un dialdéhyde en C₂-C₈ aliphatique ;
(vii) au moins un composé choisi dans le groupe constitué par les acides dicarboxyliques aliphatiques, leurs sels et anhydrides, les acides tricarboxyliques aliphatiques, leurs sels et anhydrides, les acides dicarboxyliques aromatiques, leurs sels et anhydrides, ainsi que les acides aldéhydiques, leurs sels et anhydrides ;
et un ajout comprenant un ou plusieurs des composants (iv) à (v) :
(iv) du soufre ;
(v) du sulfate d'ammonium ;
la proportion en poids du composant (i) étant > 50 % en poids et la proportion en poids de la somme des composants (ii), (iii) et (vii) dans la composition étant < 1 % en poids, et la proportion en poids de la somme des composants (iv) et (v) dans la composition étant < 50 % en poids.

2. Composition particulaire selon la revendication 1, contenant :
(i) de l'urée ;
et un additif comprenant le composant (ii) et un ou deux des composants (iii) et (vii) :
(ii) une combinaison de polyéthylène-imine et de polyvinylamine ;
(iii) de l'éthane-dial et/ou du glutaraldéhyde ;
(vii) au moins un composé choisi dans le groupe constitué par l'acide oxalique, l'acide succinique, l'acide citrique, l'acide phtalique, l'anhydride de l'acide phtalique, l'acide glyoxylique et leurs sels,
et un ajout comprenant un ou plusieurs des composants (iv) à (v) :
(iv) du soufre ;
(v) du sulfate d'ammonium ;
de préférence, la proportion en poids du composant (i) étant > 50 % en poids et la proportion en poids de la somme des composants (ii), (iii) et (vii) dans la composition étant < 1 % en poids, et la proportion en poids de la somme des composants (iv) et (v) dans la composition étant < 50 % en poids.

3. Composition particulaire selon l'une quelconque des revendications 1 à 2, dans laquelle la polyéthylène-imine du composant (ii) présente un poids moléculaire dans la plage allant de 500 à 2 000 000 Da.

4. Composition particulaire selon l'une quelconque des revendications 1 à 3, dans laquelle la polyvinylamine du composant (ii) présente un poids moléculaire dans la plage allant de 500 à 1 000 000 Da.

5. Composition particulaire selon l'une quelconque des revendications 1 à 4, **caractérisée en ce que** la proportion en poids des composants (ii), (iii) et (vii) dans la composition est < 0,5 % en poids, de préférence < 0,4 % en poids, de manière particulièrement préférée < 0,3 % en poids, et de manière tout particulièrement préférée < 0,25 % en poids.

6. Utilisation d'un additif et/ou d'un ajout tels que définis dans l'une quelconque des revendications 1 à 5 pour la fabrication d'une composition particulaire contenant de l'urée.

7. Procédé de fabrication d'une composition particulaire contenant de l'urée, comprenant les étapes suivantes :
(A) la préparation d'une solution contenant de l'urée ;
(B) la granulation ou le grelonage de la solution contenant de l'urée avec ajout d'un additif et/ou d'un ajout tels que définis dans l'une quelconque des revendications 1 à 5.

8. Procédé selon la revendication 7, dans lequel la teneur en urée de la solution est > 10 % en poids, de préférence > 50 % en poids.

9. Procédé selon l'une quelconque des revendications 7 ou 8, dans lequel la solution contenant de l'urée est granulée à l'étape (B) par granulation en lit fluidisé, comprenant les étapes suivantes :
(B1) la préparation de germes contenant de l'urée ;
(B2) la fluidisation des germes contenant de l'urée ;
(B3) la pulvérisation de la solution contenant de l'urée sur les germes en utilisant un additif et/ou un ajout définis tels que définis dans l'une quelconque des revendications 1 à 3.

10. Procédé selon l'une quelconque des revendications 7 à 9, dans lequel la température de la solution contenant de l'urée est > 120 °C, de préférence > 125 °C, de manière particulièrement préférée > 130 °C.

11. Procédé selon l'une quelconque des revendications 7 à 10, comprenant l'étape (C) :
(C) la séparation de la composition d'urée particulaire après sa fabrication est séparée en trois fractions,
une fraction (F1) contenant des particules de la taille cible souhaitée,
une fraction (F2) contenant des particules d'une taille supérieure à la taille cible souhaitée, et
une fraction (F3) contenant des particules d'une taille inférieure à la taille cible souhaitée.

12. Procédé selon l'une quelconque des revendications 7 à 11, comprenant l'étape (D) :
(D) le lavage par voie humide.

13. Utilisation d'une composition d'urée particulaire selon l'une quelconque des revendications 1 à 5 en tant qu'engrais.
